# EUROPEAN PATENT SPECIFICATION

(11) **EP 2 828 654 B1**
(45) Date of publication and mention of the grant of the patent: **06.12.2017**
(21) Application number: 13721405.2
(22) Date of filing: 22.03.2013
(51) Int. Cl.: G01N 31/22, G01N 21/78, A61B 5/08, B05D 1/18, G01N 21/64, G01N 21/77, G01N 21/81, G02B 6/036, G01N 33/18

(54) **Method and device for sensing humidity with reversible molecular dimerization and method for preparing the device**
Verfahren und Vorrichtung zum Nachweis von Feuchtigkeit mit reversibler molekularer Dimerisierung und Verfahren zur Herstellung der Vorrichtung.
Procédée et dispositif pour la détermination d'humidité avec dimérisation moléculaire réversible et procédée pour la préparation du dispositif.

(30) Priority: 23.03.2012 WO PCT/IB2012/051393
(43) Date of publication of application: 28.01.2015
(73) Proprietor: École Polytechnique Fédérale de Lausanne (EPFL), 1015 Lausanne (CH)
(72) Inventor: HORVÁTH, Endre, CH-1007 Lausanne (CH); FORRÓ, László, CH-1024 Ecublens (CH); MAGREZ, Arnaud, F-74440 Mieussy (FR); REBERNIK RIBIC, Primoz, Sl-5290 Sempeter pri Gorici (SI)
(74) Representative: Grosfillier, Philippe
(86) International application number: PCT/IB2013/052293
(87) International publication number: WO 2013/140378

(56) References cited:
- RYOTA SHINOZAKI ET AL: "Humidity-Dependent Reversible Aggregation of Rhodamine 6G Dye Immobilized within Layered Niobate K 4 Nb 6 O 17", LANGMUIR, vol. 20, no. 18, 1 August 2004 (2004-08-01), pages 7583-7588, XP055072481, ISSN: 0743-7463, DOI: 10.1021/la049354k
- SOHRABNEZHAD ET AL: "New methylene blue incorporated in mordenite zeolite as humidity sensor material", MATERIALS LETTERS, NORTH HOLLAND PUBLISHING COMPANY. AMSTERDAM, NL, vol. 61, no. 11-12, 13 April 2007 (2007-04-13), pages 2311-2314, XP022027242, ISSN: 0167-577X, DOI: 10.1016/J.MATLET.2006.09.006
- RYOKA MATSUSHIMA ET AL: "Humidity-Sensitive Color Changes of Ionic Dyes in Solid Thin Film of Sugar Gel", CHEMISTRY LETTERS, 1 January 2002 (2002-01-01), pages 436-437, XP055072486,
- ZANJANCHI M A ET AL: "Evaluation of methylene blue incorporated in zeolite for construction of an optical humidity sensor", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 105, no. 2, 28 March 2005 (2005-03-28), pages 502-507, XP004786105, ISSN: 0925-4005, DOI: 10.1016/J.SNB.2004.07.009
- HARRY H. GAO: "Tapered fiber tips for fiber optic biosensors", OPTICAL ENGINEERING, vol. 34, no. 12, 1 December 1995 (1995-12-01), page 3465, XP055072661, ISSN: 0091-3286, DOI: 10.1117/12.215379

## Description

### TECHNICAL FIELD

The present invention relates to the measurement of humidity.

The present invention may be used for medical, industrial or environmental applications.

### BACKGROUND OF THE INVENTION

The physico-chemical process of light conversion is yet under intensive investigation in order to develop more efficient devices for artificial photosynthesis, water splitting and photovoltaic applications.¹ The photo-processes of these systems are induced by nanostructured functional materials light sensitized by dye molecules adsorbed on their surface.² The quantum yield of light conversion drastically depends on the absorption spectrum of the dye. Metachromasy, being the change of the absorption spectrum of dyes without modification of the structural skeleton of the molecule, can be the result of unexpected order/disorder in the assembly of the dye molecules as well as the result of molecular conformation modifications when adsorbed on the functional material surface.^{3,4} Although metachromasy can be detrimental for device performance, possible factors influencing the absorption spectrum of the dye are largely unexplored.

Titanium dioxide based compounds are the most widely used functional materials in light conversion applications despite numerous promising alternative materials that are being explored.^{5,6} In addition to isotropic nanoparticles, titanate nanowires can be used to prepare novel 3D quasi single crystalline structures exhibiting large porosity.

This material is very efficient when used as a photoanode in solid state dye sensitized solar cells (ss-DSSCs) as it leads to a considerable enhancement of the cell efficiency.⁷

The performances of the ss-DSSC could be further improved by a more detailed investigation of the dye interaction with the titanate nanowires. In particular, a comprehensive understanding of the aggregation dependent metachromasy of dye molecules on these functional materials is necessary in order to develop more favorable coatings for efficient light conversion. This matter has not yet received appropriate attention in the case of nanowires as well as for conventional isotropic nanoparticles. Hence the effect of environmental factors, in particular humidity, has not been investigated.

Methylene Blue (MB), a cationic dye belonging to phenothiazine compounds and showing a deep blue color, is a sensitizer in photogalvanic cells for solar energy conversion.⁸ Deviation from the Beer-Lambert law (*i.e.* quantitative evolution of the transmitted visible light through the MB solution) has been ascribed to molecular aggregation.⁹ In diluted aqueous solutions, the dye forms face-to-face dimers, while increasing the dye concentration causes higher aggregates to appear. Similar aggregation associated with metachromasy occurs upon MB adsorption onto organic materials¹⁰ and onto inorganic solids.^{11,12,13,14,15,16} This effect is used for biological tissue staining or to differentiate polymorphism of clay minerals.¹⁷ A humidity dependent metachromasy of MB has already been observed but it was discussed on the basis of dye protonation¹⁸, and this study left a lot of room for further investigations.

Humidity sensors could make use of MB metachromasy. So far, efficient devices have not been realized yet. The development of low-cost sensors with fast response time and high sensitivity over a wide humidity range is very much required. For instance, the control of humidity is indispensable in air conditioning systems, during chemical processes in textile and pharmaceutical industries, for nutritional product manufacturing and civil engineering. In recent years, optical fiber based humidity sensors (OFHS) have attracted increasing attention due to their inherent features such as immunity to electromagnetic interference, the simplicity of miniaturization and the possibility of real time monitoring and remote sensing. In an OFHS, the sensitive reagents are immobilized within a solid matrix, which coats the endface of the optical fiber. Different organic and inorganic sensitive reagents, such as crystal violet, rhodamine B¹⁹ and cobalt chloride have been used.^{20,21} The solid substrate needs to fulfill numerous specifications including large porosity, mechanical stability and needs to ensure irreversible reagent immobilization to impede reagent leaching during device operation. Thus, the number of appropriate matrix materials is limited to inorganic thin films of silica²², protonated mordenite zeolite¹⁸, and organic films based on hydroxypropyl cellulose, gelatin²³ or polymethyl methacrylate.²⁴

### GENERAL DESCRIPTION OF THE INVENTION

The present invention concerns the use of reversible dimerization of a cationic dye for sensing humidity.

The cationic dye is selected from the group consisting of methylene blue, rhodamine 6G, basic green or auramine O. Preferably, the cationic dye which is used is methylene blue. In the present invention a titanate nanowire film is coated by a cationic dye. The present invention is defined in claim 1. In a preferred embodiment the humidity sensor of the present invention is provided as, for instance an optical fiber humidity sensor, for detecting humidity comprising optical fibers having a zone, said zone being defined by a material sensitive to humidity, said material comprising a cationic dye, said sensor using the reversible dimerization of the cationic dye for sensing humidity.

The cationic dye used in the sensor according to the invention may be selected from the group consisting of methylene blue, rhodamine 6G, basic green or auramine O. Preferably, the cationic dye is methylene blue.

Another object of the invention is to provide a process for manufacturing a sensor according to the invention, comprising the following steps:
a. Depositing a layer of a cationic dye on a film of titanate nanowires,
b. Providing optical fibers,
c. Depositing the coated film of step a on said optical fibers.

The coated film of step a may be deposited by dip coating.

The present invention also concerns a method for detecting and measuring humidity comprising the following steps:
a. Providing a sensor according to the invention,
b. Exposing the sensor to humidity,
c. Determining the intensity variation of the light absorbance of the cationic dye.

Another object of the invention is the use of the sensor according to the invention in a breathing monitoring system for detecting hyperventilation.

### DETAILED DESCRIPTION OF THE INVENTION

In the following, the present invention will be described in detail using figures and examples.

### BRIEF DESCRIPTION OF THE FIGURES

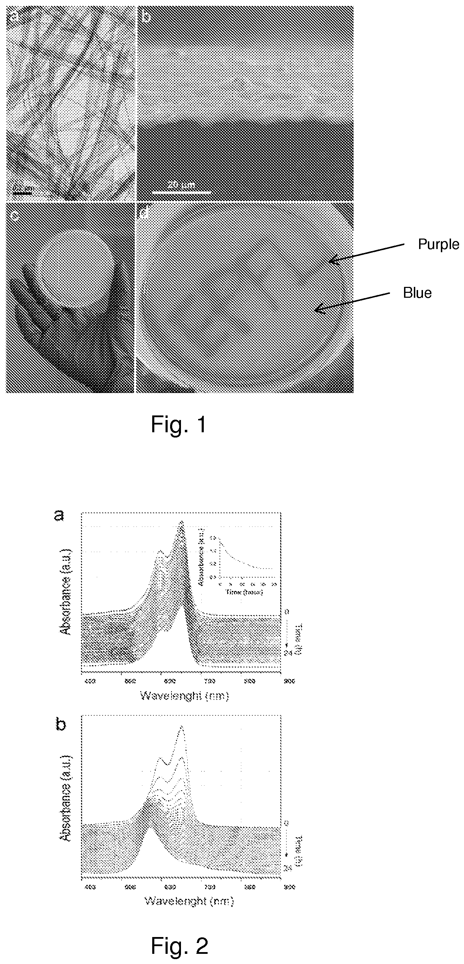

**FIG. 1** represents characteristic TEM, SEM images of the component materials and photo of the humidity sensing principle: (a) as-synthesized trititanate nanowires, (b), (c) titanate nanowire based thin film on glass substrate (d) MB sensitized titanate nanowire thin film showing the blue to purple color change in places where exposed to high humidity.
**FIG. 2** illustrates visible (Vis) spectral changes of MB (metachromasy) on surface of protonated titanate nanowire based thin films in aqueous solution. (a) Quantitative adsoption; absorbance of MB in aqueous phase (b) Qualitative adsorption; absorbance of MB on the surface of the nanowires.

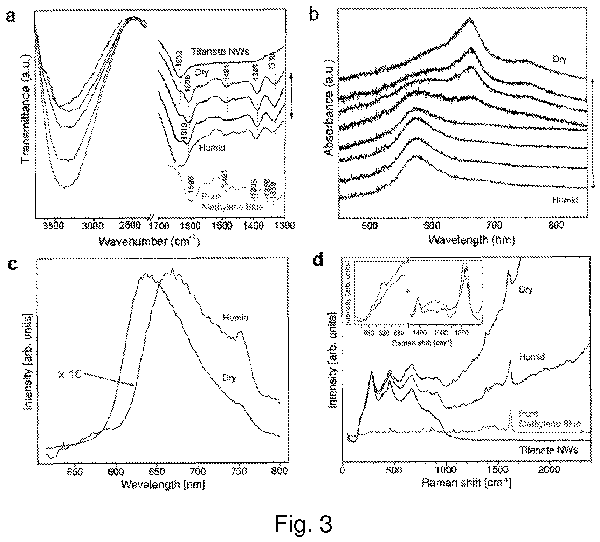

**FIG. 3** shows (a) FTIR spectra of MB (metachromasy) adsorbed onto the titanate nanowire surface upon drying, (b) VIS spectral changes of MB (metachromasy) adsorbed onto the nanowire surface upon drying, (c) Laser induced (488 nm excitation) fluorescence spectra of MB-nanowire materials in the humid and dry states. (d) Raman spectra of MB-nanowire materials in dry and humid states, pure MB and pure nanowires (inset: zoom of the spectral regions for dry and humid MB-nanowire materials showing the peaks corresponding to the C-S-C skeletal deformation and C-C ring stretching vibrations of MB - see text; the fluorescence background was subtracted). The excitation wavelength was 488 nm. The fluorescence background present in the Raman spectra of MB sensitized titanate nanowires and absent for pure MB crystals suggests that some MB monomers can remain present on the surface of TiONW even in the humid state of the sample.

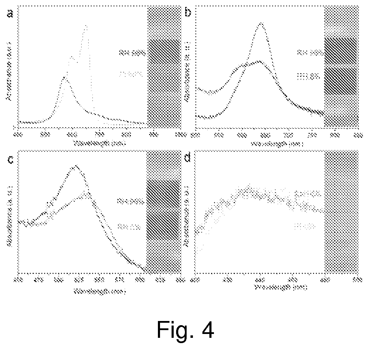

**FIG. 4** represents humidity dependent metachromasy of different dyes on titanate nanowire surface exposed to low and high relative humidity (RH) atmosphere: (a) methylene blue, (b) basic green, (c) rhodamine 6G, (d) auramine O.

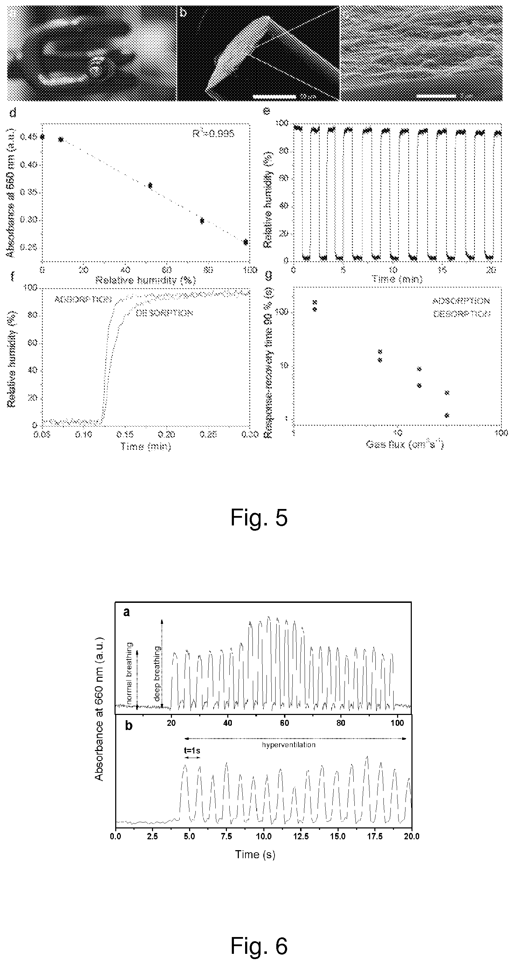

**FIG. 5** shows (a) Photo, SEM images of MB-TiONW optical fiber humidity sensor (b) fiber end (c) porous titanate nanowire thin film on the top of the fiber. Variation of absorbance with respect to the relative humidity (RH) measured at 660 nm (d) calibration curve, (e) repeated cycles by alternating exposure of the sensor to streams with 0 % and 98% RH, (f) representative response and regeneration signal obtained at the highest flux used (30 cm³/s), (g) response time of the sensor against the air flux over the sensor.
**FIG. 6** illustrates a variation of absorbance with respect to human inhale and exhale modes (a) normal and deep breathing, (b) increased respiratory rate, e. g. hyperventilation.

### EXAMPLES

### Materials

Titanium dioxide (anatase, TiO₂), sodium hydroxide (NaOH), methylene blue (MB) and hydrochloric acid (HCl) pure grade, were obtained from Sigma-Aldrich and were used without further purification. Standard pH buffer solutions were obtained from Metrohm. Microscopic glass slides for film preparation were purchased from Menzel GmbH & Co KG.

### Preparation of protonated titanate nanowires and their suspension

Protonated titanate nanowires were prepared by a two-step hydrothermal process. The details about the typical synthesis procedure are given elsewhere.7⁷

To prepare nanowire suspensions TiONW powder (2.4 g) is dispersed in 100 mL of 2-propanol (99.8% Merck), and the mixture is homogenized with an ultrasonic tip (Bandelin Sonopuls, Microtip MS73) at 10% amplitude for 30 min.

Thin films were fabricated on microscopic glass substrates. The nanowire solution of 24 mgmL⁻¹ in isopropyl alcohol is doctor bladed using a 40 µm spacer (3M) to obtain the desired film thickness of 2.5µm. Once deposited onto glass slides the samples are heat treated at 150 °C in air for 2 h in order to evacuate the solvent.

### UV-VIS spectroscopy

All adsorption spectra were recorded using a CARY 50 UV-VIS Spectrophotometer in the wavelength range 500-800 nm. The matched quartz cuvettes of 0.5 cm pathlength were used. The MB solution concentration was chosen such that the absorbance values did not exceed 1.5. All the measurements reported in this work were carried out at 25±1 °C. The cationic dye (MB) was used without further purification, hence its spectrum in water had a similar shape to that reported in the literature with λₘₐₓ=664±1 nm. For the qualitative adsorption kinetics, 1 cm² pieces of glass substrates holding 2.5 µm thick films of protonated titanate nanowires were placed into the cuvettes so that the light passed through the film. Subsequently 1.5 mL of 20 mgL⁻¹ MB solution was added and the spectra were recorded at 60 min time intervals for 24 hours. The quantitative adsorption measurements were done by placing the thin film on the sides of the cuvettes so that the light did not pass through the film, only the aqueous MB solution. The residual MB concentrations in the supernatant solutions were determined. The amount of MB adsorbed onto TiONWs was computed by the difference between the initial MB concentration and the residual concentration.

The obtained samples were first thoroughly rinsed with distilled water to remove nonadsorbed MB and then dried at 100 °C for 1 h to obtain the MB-TiONW nanocomposite. In order to determine the MB desorption kinetics, the samples were subjected to different solvents like distilled water, ethanol and tetrahydrofuran.

### FTIR spectroscopy

The FTIR spectra for all the samples were measured using a Nexus Nicolet FTIR spectrometer at frequencies ranging from 400-4000 cm⁻¹.

### Raman and laser induced fluorescence spectroscopy

Measurements were performed on a MicroHR spectrometer (HORIBA Jobin Yvon) with a spectral resolution of ∼8 cm⁻¹. The 488 nm line of an Ar ion laser (Koheras) was used as an excitation source. Spectra were recorded in a backscattering geometry using a long working distance objective (20X Mitutoyo Plan Apo SL Infinity-Corrected). Rayleigh scattered light was filtered out by placing a holographic notch filter (Kaiser Optical Systems) in front of the spectrometer entrance slit.

For Raman and fluorescence measurements on solid samples, a rotating sample holder was used in order to minimize the effects of photobleaching. The 0% relative humidity environment (dry state) was obtained by exposing the sample surface to a high nitrogen flux. For measurements at 100% relative humidity (humid state) a drop of water was placed onto the sample surface. The excess water was then removed and a cover slip was placed onto the sample to prevent evaporation due to high laser fluxes.

All of the spectra were acquired at room temperature. For Raman measurements, several acquisitions were necessary in order to filter out cosmic spikes. The pure TiONW and MB-TiONW Raman spectra were normalized to the TiONW peak at 280 cm⁻¹. The pure MB Raman spectrum was normalized to the 1625 cm⁻¹ peak of MB-TiONW.

For fluorescence measurements on liquid samples a quartz cuvette was used. The laser was focused near the surface of the liquid.

### Results and discussion

Here we first investigate the dye metachromasy mechanism of MB molecules adsorbed on protonated titanate nanowire-based thin films. The color change caused by molecular dimerization is induced by water adsorption. This reveals that humidity is a key parameter that needs to be controlled in order to optimize the light conversion performances of the devices. We then took advantage of the humidity dependent metachromasy to develop a novel humidity sensor based on an optical fiber. The system exhibits high sensitivity, excellent reversibility and fast response time. The implementation of the sensor in breathing monitoring systems is also presented.

Fig. 1 shows representative nanowires produced by the hydrothermal process. They are uniform in size with a mean diameter of about 60 nm and a length in the 1-5 µm range. They crystallize in a trititanate structure.²⁵ The nanowires can be homogeneously deposited on substrates by doctor blading using isopropanol as solvent (Fig. 1b). During the deposition, the nanowires first self assemble in a quasi two-dimensional network, parallel to the substrate surface.

During isopropanol evaporation, the nanowire density increases. However, the final porosity of the dried film remains about 40% larger than that of films made of isotropic particles.⁷ This is a result of the low packing density of the nanowires due to their large anisotropy (Fig. 1a). This large open porosity offers efficient molecule diffusion into the titanate architecture, resulting in homogenous adsorption of dyes onto the nanowire surface. Consequently, homogenous blue films are obtained when dipped in an aqueous solution of methylene blue (Fig. 1d).

The spectrophotometric analysis of the film during MB adsorption reveals visible spectral changes of MB on surface of protonated titanate nanowire based thin films in aqueous solution. Depending on the experimental geometry, qualitative and quantitative information can be obtained. The concentration of MB in water was chosen as such where aggregation does not exceed dimerization. Fig 2a shows the absorbance of MB in aqueous phase. The spectrum of the free MB in aqueous solution displays a strong absorbance at 664 nm and a shoulder at 610 nm, ascribed to the characteristic bands of the MB monomer in solution. Increase of the contact time leads to adsorption of MB onto titanate nanowires. As expected, the absorbance values of the monomeric species decreased with increasing contact time, due to dye adsorption onto the solid nanowire surface. The position of the band maxima remains the same. The quantitative adsorption study shows that more than 85mg of MB can be adsorbed per gram of titanate nanowires (Fig. 2a and inset). This corresponds to a monolayer coverage of MB on the nanowire surface (1 molecule/nm²). Because of the applied experimental geometry the absorption signal in Fig. 2b corresponds to the superposition of absorption signals from MB molecules in solution and MB on the surface of the nanowires, therefore only qualitative information can be deduced. The intensity of the 664 nm peak decreases, while the blue shift of the 610 nm peak indicates MB aggregation. In addition, a low intensity absorption band centered at 790 nm appears. It reveals the presence of Brønsted acid states converting adsorbed MB dyes into protonated forms like MBH²⁺ and MBH₂³⁺.²⁶ The decrease of the 664 nm peak intensity as well as the blue shift of the 610 nm peak in the UV-Vis spectra points to a molecular aggregation of MB with increasing exposure time of the titanate film to the MB solution.

After 24 hours, the bands of MB dimers dominate the spectrum (Fig. 2b). However, the MB sensitized titanate film undergoes a drastic metachromasy from purple to blue upon drying (Fig. 1d).

This color change in the UV-Vis spectrum is associated with the disappearance of the peak representative of MB dimers while the monomer adsorption bands emerge (Fig. 2b). This effect is reversible. The film turns back to purple as it is exposed to ambient humidity. On the basis of the fluorescent behavior of MB molecules dissolved in aqueous solution (see supporting information), the humidity dependence of the fluorescence intensity corroborates the UV-Vis observations. It substantiates that the dye metachromasy induced by humidity originates from MB molecule dimerization (Fig. 3c). In addition, the blue-shift of about 50 nm and 20 nm of the fluorescence peak of MB adsorbed on titanate nanowires in the dry and humid states respectively when compared to the fluorescence peak of the dissolved MB molecules confirms the interactions of the molecules with the titanate nanowire surface. The weaker blue-shift of the fluorescence in the humid state is very likely a result of reduced interactions of MB with the nanowire film as well as MB dimerization.

Molecular orientation of MB in the dry and humid state was studied by means of Raman scattering and FTIR spectroscopy. In the high frequency part of the Raman spectrum, the splitting of the 1620 cm⁻¹ peak (into two components centered at 1610 cm⁻¹ and 1626 cm⁻¹) was assigned on the basis of a thionine reference spectrum to C-C ring stretching vibrations of the MB molecule (Fig. 3d and inset).²⁷ The low frequency component (1610 cm⁻¹) is much stronger for MB in the dry as compared to the humid state, where the peak shape is closer to the one of the spectrum recorded for pure MB crystals. The 1620 cm⁻¹ band splitting was previously observed only by surface enhanced Raman spectroscopy (SERS).²⁸ Although the surface selection rules for metals are different than for oxide surfaces, the high intensity of the mode at 1610 cm⁻¹ in the dry state suggests a signal enhancement mechanism resulting from the interaction of the MB molecular π-orbitals with the electronic structure of the titanate nanowire surface. Therefore, the most likely adsorption configuration of MB molecules in the dry state is in the plane parallel to the nanowire surface. In humid atmosphere, water adsorption results in weaker MB interactions with the titanate nanowires. Molecules are tilted as compared to the dry state configuration. This leads to a decrease in the intensity of the 1610 cm⁻¹ Raman component. The spectrum in the humid state resembles the one of pure MB crystals.

The above conclusions are consistent with the low frequency part of the Raman spectra (Fig. 3d and inset). Despite the strong contributions from titanate nanowires, a clear peak at 618 cm⁻¹ appears only in the dry state. Although the relative intensity of this peak is low as it coincides with the Raman features of titanate nanowires, the data were reproduced on several samples. It is assigned in SERS on metal substrates to the C-S-C skeletal deformation of MB molecules.²⁹ The increase in intensity and the slight upshift (∼10 cm⁻¹) of this peak with respect to the pure MB spectrum might be an indication that the adsorption of MB involves the S atom which is in addition consistent with the structural steric hindrance of the molecule when the adsorption configuration is parallel to the nanowire surface. The C-S-C vibration mode vanishes in the humid state suggesting that molecules undergo orientational changes with increasing relative humidity. In the FTIR spectra (Fig. 3a), the downshift of the ring stretching mode from 1610 to 1605 cm⁻¹ upon drying further indicate that the π-electron network of MB molecules must be perturbed during the adsorption configuration change.

From these spectroscopic analyses, a mechanism for the humidity dependence of metachromasy can be proposed (Fig. 3). The orientational change of the MB molecules is driven by the adsorption competition between the amphilic MB and the water molecules on the hydrophilic surface of the nanowires. In the dry state, the electrostatic interaction between the cationic MB and the negatively charged surface of the titanate nanowires holds the dye molecules parallel to the nanowire surface.³⁰ When the film is exposed to humidity, the water affinity of the nanowires as well as the stacking affinity between π-systems overcomes the MB-titanate electrostatic interactions. Water molecules force the MB to undergo an orientational change towards MB dimers standing on their edges on the nanowire surface. This induces a modification of the light absorption properties of the MB dye from blue to purple.

This humidity induced metachromasy is observed for other dye molecules including rhodamine 6G, basic green and auramine O (Fig. 4). The control of the molecular aggregation behavior is a necessity in order to optimize the sensitization efficacy of the dye. Metachromasy can be detrimental for the performances of light converting devices. However, we took advantage of the observed reversibility of this effect to build humidity sensors based on optical fibers. For the OFHS fabrication, a film of titanate nanowires previously coated with a monolayer of MB molecules is deposited by dip coating on optical fibers having a core and cladding diameters of 62.5 and 125 µm, respectively (Fig. 5a, b). Interestingly, nil desorption of MB was observed in solvents like water, ethanol or tetrahydrofuran to name but a few. From the technological point of view, this enables future integration of the films in devices using a wide range of substrates including water soluble materials or the measurement of water concentration in non aqueous solvents. Red light (λ=660 nm) from a light emitting diode (LED) is guided to the input of the optical fiber. The working principle of the OFHS is based on the intensity variation of the 660 nm band (absorption induced by MB monomers) when the sensor is exposed to different humidity levels. In dry conditions, MB monomers absorb the light while an increase of the relative humidity, resulting in MB dimerization, reduces light absorbance.

Consequently, the intensity of the transmitted light at 660 nm increases. Absorbance variation of the OFSH with respect to different relative humidity environments was measured in the 0-98% range (Fig. 5d). However, the sensor response is linear versus RH between 8 and 98%. Deviation from linearity of the response is caused by the technical difficulty to achieve a 0% RH atmosphere in a UV-Vis spectrometer cuvette used for this study. Measured values in completely dried atmospheres always corresponded to slightly higher RH % causing a small deviation from linearity below 8% RH. The sensor signal shows excellent reproducibility and reversibility with very little hysteresis when the RH is repeatedly changed between 8 and 98% (Fig. 5e, f). Other environmental parameters which modify the water adsorption-desorption equilibrium on solids affects the signal shape and intensity. For example, at constant temperature, the signal recovery and response time of our sensor on log-log scale depends linearly on the air flow over the sensitized film (Fig. 5g). For a 30 cm³min⁻¹ flow, the time to reach 90% of the signal is reduced to 1s while it remains lower than 100s for a 1 cm³min⁻¹ flow.

These results confirm that our device, based on titanate nanowires sensitized with MB, perform better than existing technology. It shows faster response time over a broader RH range. These performances are the result of a reduced film thickness associated with abundant macroporosity. These favorable characteristics allow rapid and homogeneous water diffusion through the entire sensitized film yielding a reduced hysteresis and fast response time of the OFHS.

This technology could be used in monitoring the moisture in any environment where the use of electronics is forbidden such as flammable liquid tanks or industrial gas pipelines.

Taking advantage of the fast response, the OFHS can be used to record human breathing modes, being the first monitoring device based on dye metachromasy. In our experiment, the sensing endface part was connected to an external tubing system. This way both inhaling and exhaling can be monitored. The respiratory rate of a healthy adult male human counted using an 80 seconds count period was recorded. During the examination the person was asked to take several deep breaths as well (Fig. 6a). The higher intensity of the signal indicates that after an appropriate calibration the sensor could reflect the volume of the air inhaled or exhaled from the lungs. With fast response time (0.5 s), the OFSH can monitor an increase of breathing frequency, as well. Therefore, it fulfils the requirements to detect hyperventilation which could be first indications of respiratory dysfunction (Fig. 6b).

### Conclusions

Spectroscopic study of MB adsorption on the surface of titanate reveals that the metachromasy observed upon exposure to humidity is related to a change of the MB molecular configuration. In the dry state, MB molecules are held parallel to the surface of the titanate nanowires by electrostatic interactions. In a wet atmosphere, the hydrophilicity of the titanate nanowires, associated to the stacking affinity of the dyes, forces MB to form dimers standing on the nanowire surface. The dimerization and change of the molecular configuration result in a reversible MB metachromasy which was used in optical fiber humidity sensors and in breathing monitoring devices. The existence of metachromasy for other dyes reveals that a detailed characterization of dye molecular configuration is needed (although it is not much studied) since it affects sensitization efficacy and consequently the performances of devices using dye sensitized functional materials like solar cells.

### REFERENCES

1. D. Gust, T. A. Moore, A. L. Moore, Acc. Chem. Res., 2001, 34, 40.
2. S. Ferrere, A. Zaban, B. A. Gregg, J. Phys. Chem. B, 1997, 101, 4490.
3. B. Sylven, Quarterly Journal of Microscopical Science, 1954, 95, 327.
4. W. C. Holmes, Stain Technology, 1926, 1, 116.
5. A. L. Linsebigler, G. Lu, J. T. Yates, Chem. Rev.,1995, 95, 735.
6. J.-J. Lee, Md. M. Rahman, S. Sarker, N.C. Deb Nath, A.J. Saleh Ahammad, J. K. Lee,. in Advances in Composite Materials for Medicine and Nanotechnology, (Ed. B. Attaf), ISBN: 978-953-307-235-7, InTech, 2011.
7. N. Tétreault, E. Horváth, T. Moehl, J. Brillet, R. Smajda, S. Bungener, N. Cai, P. Wang, P. S. M. Zakeeruddin,; L. Forró, A. Magrez, M. Grätzel, ACS Nano, 2010, 4, 7644.
8. W. J. Albery, Acc. Chem. Res., 1982, 15, 142
9. K. Bergmann, C.T. O'Konski, J. Phys. Chem., 1963, 67, 2169.
10. R. Matsushima, H. Okuda, M. Aida, A. Ogiue, Bull. Chem. Soc. Jpn. 2001, 74, 2295.
11. V. Ramamurthy, D. R. Sanderson, D. F. Eaton, J. Am. Chem. Soc. 1993, 115, 10438.
12. S. M. Ohline, S. Lee, S. Williams, C. Chang, Chem. Phys. Letters, 2001, 346, 9.
13. H. Nishikiori, S. Nagaya, N. Tanaka, A. Katsuki, T. Fujii, Bull. Chem. Soc. Jpn. 1999, 72, 915.
14. N. Matsuda, J. Zheng, D-K. Qing, A. Takatsu, K. Kato, Applied Spectroscopy, 2003, 57, 100.
15. K. Adachi,; T. Mita, T. Yamate, S. Yamazaki, H. Takechi, H. Watarai, Langmuir, 2010, 26, 117.
16. J. Cenens, D. P. Vliers, R. A. Schoonheydt, F.C. De Schryver, in: Proceedings Int. Clay Conf., Denver 1985, p.352.
17. P. T. Hang, G. W. Brindley, Clay and Clay Minerals, 1970, 18, 203.
18. M. A. Zanjanchi, Sh. Sohrabnezhad, Sensors and Actuators B, 2005, 105, 502.
19. S. Otsuki, K. Adachi, T. Taguchi, Sensors and Actuators B, 1998, 53, 91.
20. T. E. Brook, M. N. Taib, R. Narayanaswamy; Sensors and Actuators B: Chem., 1997,39,272.
21. F. Boltinghouse, K. Abel, Anal. Chem., 1989, 61, 1863.
22. J. Estella, P. de Vincente, J. C. Echeverría, J.J. Garrido, Sensors and Actuators B, 2010, 149, 122.
23. A. Kharaz, B.E. Jones, Sensors and Actuators A, 1995, 46-47, 491.
24. S. Muto, H. Sato, T. Hosaka, Jpn. J. Appl. Phys., 1994, 33, 6060.
25. E. Horváth, Á. Kukovecz, Z. Kónya, I. Kiricsi, Chem. Mater., 2007, 19, 927.
26. Cenens, J.; Schoonheydt, R. A. Clay and Clay Minerals, 1988, 36, 214-224.
27. K. Hutchinson,; R. E. Hester, J. Chem. Soc., Faraday Trans. 1, 1984, 80, 2053.
28. G.-N. Xiao, S.-Q. Man, Chem. Phys. Lett., 2007, 447, 305.
29. C. Ruan, W. Wang, B. Gu, J. Raman Spectrosc., 2007, 38, 568.
30. H. Tokudome, M. Miyauchi Angew. Chem. Int. Ed., 2005, 44, 1974.

## Claims

1. Humidity sensor based on the reversible dimerization of a cationic dye, said sensor comprising a zone defined by a material sensitive to humidity that comprises a titanate nanowire film coated by a cationic dye.

2. Humidity sensor according to claim 1 including one or several optical fibers, wherein said zone is located at the tip of said fiber(s).

3. Humidity sensor according to claim 1 or 2, wherein said cationic dye is selected from the group consisting of methylene blue, rhodamine 6G, basic green or auramine O.

4. Sensor according to claim 3, wherein said cationic dye is methylene blue.

5. A process for manufacturing a sensor as defined in claims 1 to 4, **characterized by** the following steps:
a. Depositing a layer of a cationic dye on a film of titanate nanowires,
b. Providing optical fibers,
c. Depositing the coated film of step a on said optical fibers.

6. The process according to claim 5, wherein the coated film of step a is deposited by dip coating.

7. A method for detecting and measuring humidity comprising the following steps:
a. Providing a sensor as defined in claims 1 to 4,
b. Exposing the sensor to humidity,
c. Determining the intensity variation of the light absorbance of the cationic dye.

8. Use of the sensor according to claims 1 to 4 in a breathing monitoring system for detecting hyperventilation.

9. Use of the sensor according to claims 1 to 4 as a humidity indicator card.

10. Use of the sensor according to claims 1 to 4 as a security label, security sticker.

## Patentansprüche

1. Feuchtesensor auf der Grundlage der reversiblen Dimerisierung eines kationischen Farbstoffs, wobei der Sensor eine Zone umfasst, die durch ein feuchteempfindliches Material definiert ist, das einen mit einem kationischen Farbstoff beschichteten Titanat-Nanodrahtfilm umfasst.

2. Feuchtesensor gemäß Anspruch 1, der eine oder mehrere optische Fasern enthält, wobei die Zone an der Spitze der Faser(n) angeordnet ist.

3. Feuchtesensor gemäß Anspruch 1 oder 2, wobei der kationische Farbstoff ausgewählt ist aus der Gruppe bestehend aus Methylenblau, Rhodamin 6G, Malachitgrün und Auramin O.

4. Sensor gemäß Anspruch 3, wobei der kationische Farbstoff Methylenblau ist.

5. Verfahren zum Herstellen eines Sensors gemäß einem der Ansprüche 1 bis 4, **gekennzeichnet durch** folgende Schritte:
a. Aufbringen einer Schicht eines kationischen Farbstoffs auf einen Film von Titanat-Nanodrähten,
b. Bereitstellen von optischen Fasern,
c. Aufbringen des beschichteten Films aus Schritt a auf die optischen Fasern.

6. Verfahren gemäß Anspruch 5, wobei der beschichtete Film aus Schritt a durch Tauchbeschichten aufgebracht wird.

7. Verfahren zum Nachweisen und Messen von Feuchte, umfassend folgende Schritte:
a. Bereitstellen eines Sensors gemäß Ansprüchen 1 bis 4,
b. Exponieren des Sensors gegenüber Feuchte,
c. Bestimmen der Intensitätsveränderung der Lichtabsorbanz des kationischen Farbstoffs.

8. Verwendung des Sensors gemäß Ansprüchen 1 bis 4 in einem Atmungsüberwachungssystem zum Nachweisen von Hyperventilation.

9. Verwendung des Sensors gemäß Ansprüchen 1 bis 4 als Feuchteindikatorkarte.

10. Verwendung des Sensors gemäß Ansprüchen 1 bis 4 als Sicherheitsetikett, Sicherheitsaufkleber.

## Revendications

1. Capteur d'humidité basé sur la dimérisation réversible d'un colorant cationique, ledit capteur comprenant une zone définie par un matériau sensible à l'humidité qui comprend un film de nanofils de titanate revêtu d'un colorant cationique.

2. Capteur d'humidité selon la revendication 1 comprenant une ou plusieurs fibres optiques, dans lequel ladite zone est située au bout de ladite ou desdites fibres optiques.

3. Capteur d'humidité selon la revendication 1 ou 2, dans lequel ledit colorant cationique est choisi dans le groupe constitué par le bleu de méthylène, la rhodamine 6G, le vert basique et l'auramine O.

4. Capteur selon la revendication 3, dans lequel ledit colorant cationique est le bleu de méthylène.

5. Procédé pour la fabrication d'un capteur tel que défini dans les revendications 1 à 4, **caractérisé par** les étapes suivantes :
a. le dépôt d'une couche d'un colorant cationique sur un film de nanofils de titanate,
b. l'utilisation de fibres optiques,
c. le dépôt du film revêtu de l'étape a sur lesdites fibres optiques.

6. Procédé selon la revendication 5, dans lequel le film revêtu de l'étape a est déposé par revêtement par trempage.

7. Procédé pour la détection et la mesure d'humidité comprenant les étapes suivantes :
a. l'utilisation d'un capteur tel que défini dans les revendications 1 à 4,
b. l'exposition du capteur à de l'humidité,
c. la détermination de la variation d'intensité de l'absorbance de lumière du colorant cationique.

8. Utilisation du capteur selon les revendications 1 à 4 dans un système de surveillance de la respiration pour la détection de l'hyperventilation.

9. Utilisation du capteur selon les revendications 1 à 4 en tant que carte indicatrice d'humidité.

10. Utilisation du capteur selon les revendications 1 à 4 en tant qu'étiquette de sécurité, autocollant de sécurité.
